(19) Europäisches Patentamt
European Patent Office
Office européen des brevets

(11) Veröffentlichungsnummer: **0 034 363**
**B1**

(12) # EUROPÄISCHE PATENTSCHRIFT

(45) Veröffentlichungstag der Patentschrift: **27.07.83**

(51) Int. Cl.³: **C 07 J 41/00**

(21) Anmeldenummer: **81101113.9**

(22) Anmeldetag: **17.02.81**

(54) **Neue Steroidverbindung mit einer 20-Amino-Funktion und Verfahren zu ihrer Herstellung.**

(30) Priorität: **19.02.80 US 122397**
**19.02.80 US 122321**

(43) Veröffentlichungstag der Anmeldung: **26.08.81 Patentblatt 81/34**

(45) Bekanntmachung des Hinweises auf die Patenterteilung: **27.07.83 Patentblatt 83/30**

(84) Benannte Vertragsstaaten: **AT BE CH DE FR GB IT LI LU NL SE**

(56) Entgegenhaltungen: **US-A-2 566 336**

(73) Patentinhaber: **HENKEL CORPORATION, 4620 West 77th Street, Minneapolis Minnesota 55435 (US)**

(72) Erfinder: **Krbechek, Leroy O., 2041 Orkla Drive, Minneapolis Minnesota 55427 (US)**

(74) Vertreter: **Fues, Johann Friedrich, Dr. et al, Deichmannhaus am Hauptbahnhof, D-5000 Köln 1 (DE)**

**EP 0 034 363 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Neue Steroidverbindung mit einer 20-Amino-Funktion und Verfahren zu ihrer Herstellung

Die Erfindung betrifft eine neue Steroid-20-Aminoverbindung bzw. ihre Salze, die durch chemische Transformation — letztlich aus Steroidverbindungen natürlichen Ursprungs — zugänglich ist und eine wichtige Zwischenstufe bei der Herstellung von Dehydroprogesteron, Progesteron und verwandten Verbindungen insbesondere aus Steroidsubstraten natürlichen Ursprungs darstellt. Die Erfindung betrifft weiterhin das Verfahren zur Herstellung dieser neuen Steroidverbindung.

Gegenstand der Erfindung ist dementsprechend in einer ersten Ausführungsform das 20-Amino-3-oxo-pregna-1,4-dien, und zwar sowohl in Form der freien Base als in Form seiner Salze mit anorganischen oder organischen Säuren. In einer weiteren Ausführungsform ist Gegenstand der Erfindung das Verfahren zur Herstellung dieser neuen 20-Amino-Steroidverbindung, wobei dieses Verfahren dadurch gekennzeichnet ist, daß man 3-Oxo-pregna-1,4-dien-20-isocyanat und/oder 3-Oxo-pregna-1,4-dien-20-tert.-butylcarbamat hydrolytisch spaltet.

Die Strukturformel der neuen Verbindung ist wie folgt:

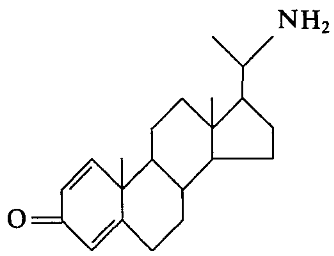

wobei in den entsprechenden Salzen anstelle der Aminogruppe $-NH_2$ in 20-Stellung die Salzgruppierung $-NH_2 \cdot HY$ vorliegt, worin HY eine anorganische oder organische Säure darstellt.

Die Herstellung der neuen Verbindung und ihre Bedeutung im Rahmen der Gewinnung von Dehydroprogesteron, Progesteron und verwandten Verbindungen aus Steroidverbindungen natürlichen Ursprungs wird aus der folgenden Darstellung ersichtlich: Die veröffentlichte Europäische Patentanmeldung 79101036.6/004913 beschreibt u. a. ein Verfahren, bei dem 3-Oxo-pregna-1,4-dien-20-carbonsäure($\Delta^{1,4}$-BNC) durch mikrobiellen Seitenkettenabbau an 17-C-Seitenketten-Steroidsubstraten natürlichen Ursprungs gewonnen werden kann. Die Strukturformel dieser in technischem Maßstab aus Sterinverbindungen pflanzlichen oder tierischen Ursprungs, wie Cholesterin, Sitosterin, Stigmasterin u. dgl., wohlfeil erhältlichen Verbindung entspricht dem Formelbild A

A

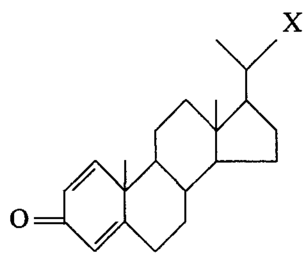

wobei X der Carboxylgruppe $-COOH$ entspricht. Zur Umwandlung dieses Abbauprodukts zu Verbindungen des Progesteron- bzw. Dehydroprogesteron-Typs muß der Substituent X in 20-Stellung zur 20-Oxogruppe umgewandelt werden. Hierzu bedarf es des Abbaus der Carboxylgruppe in dieser Stellung und der Umwandlung von C-20 zur Oxogruppe.

In der Europäischen Patentanmeldung 81100145.2/34248 wird ein mit hohen Ausbeuten arbeitendes Verfahren zur Umwandlung der $\Delta^{1,4}$-BNC in ihr Säurehalogenid, insbesondere das entsprechende $\Delta^{1,4}$-BNC-Säurechlorid beschrieben. Die Umwandlung erfolgt durch Behandlung der $\Delta^{1,4}$-BNC mit insbesondere Thionylchlorid bei niederen Temperaturen mit im wesentlichen stöchiometrischen Mengen der Reaktanten. In der formelmäßigen Darstellung entspricht dieses Säurehalogenid dem Formelbild A mit der Bedeutung von $-COCl$ für X.

In der parallelen Europäischen Patentanmeldung 81101114.7/34364 (»Neue 20-substituierte 3-Oxo-pregna-4-en-steroidverbindungen und Verfahren zu ihrer Herstellung« mit U. S. Priorität vom 19. Februar 1980, Aktenzeichen 122 397) wird die Umwandlung dieses Säurehalogenids zum entsprechenden Säureamid bzw. Carboxamid beschrieben und beansprucht (X im Formelbild $A = -CONH_2$). Die Reaktion erfolgt durch Umsetzung des Säurehalogenids mit Ammoniak oder einer Ammoniak liefernden Verbindung, wie Ammoniumhydroxid, in wenigstens stöchiometrischen Mengen. Gegenstand dieser zuletzt genannten Europäischen Patentanmeldung ist weiterhin die

Umwandlung dieses Säureamids bzw. Carboxamids unter gleichzeitiger Umlagerung zum Isocyanat (X im Formelbild A= —NCO), gewünschtenfalls unter gleichzeitiger oder getrennter weiterführender Umsetzung mit einem einwertigen Alkohol zum entsprechenden Steroid-20-Carbamat (X im Formelbild A= —NHCOOR). Die Umwandlung des Säureamids zum Isocyanat erfolgt gemäß der Anweisung dieser Europäischen Patentanmeldung durch Umsetzung des Steroid-20-Säureamids mit Bleitetraacetat in wenigstens äquivalenten Mengen unter vorzugsweise wenigstens weitgehend wasserfreien Bedingungen und zweckmäßig unter Inertgas. Wird die Umsetzung in einem aprotischen Lösungsmittel durchgeführt — beispielsweise in halogenierten Kohlenwasserstoffen —, so gelingt die Isolierung des 3-Oxo-pregna-1,4-dien-20-isocyanats. Das Isocyanat kann seinerseits mit einwertigen Alkoholen, insbesondere mit geradkettigen und/oder verzweigten Alkanolen, durch Alkoholaddition an die Isocyanatgruppe zum Steroid-20-Carbamat umgewandelt werden. Als brauchbar ist u. a. tert.-Butanol als alkoholischer Reaktant herausgestellt. Die Gewinnung des Steroid-20-Carbamats kann allerdings auch in einem Zuge aus dem entsprechenden Säureamid derart erfolgen, daß ohne Isolierung des Isocyanats die Umwandlung zum Carbamat erfolgt.

Andere Verfahren zur Herstellung der gleichen Carbamate und insbesondere auch des 3-Oxo-pregna-1,4-dien-20-tert.-butylcarbamats sind in den parallelen Europäischen Patentanmeldungen 81101119.6/34365 (»Verfahren zur Umwandlung von Steroid-20-Carbonsäureamiden in die entsprechenden Steroid-20-Carbamate« mit U. S. Priorität vom 19. Februar 1980, Aktenzeichen: 122 395) 81101117.0/34366 (»Verfahren zur Herstellung von Steroid-20-Carbamatverbindungen« mit U. S. Priorität vom 19. Februar 1980, Aktenzeichen: 122 398) beschrieben. Gemäß den Verfahren dieser Parallelschutzrechte wird zunächst — ausgehend vom Säureamid der $\Delta^{1,4}$-BNC-Brom an den Stickstoff der Säureamidgruppe eingeführt und dann durch Abspaltung von Bromwasserstoff mit starken Basen unter Umlagerung zur Isocyanatstruktur und vorzugsweise unter gleichzeitiger Anlagerung von tert.-Butanol das entsprechende Carbamat gebildet.

Das erfindungsgemäße Verfahren zur Herstellung des 20-Amino-3-oxo-pregna-1,4-dien beruht auf der Feststellung, daß sowohl das entsprechende 20-Isocyanat (siehe Formelbild A mit X= —NCO) als auch das entsprechende tert.-Butylcarbamat (Formelbild A mit X= —NHCOO-tert.-Butyl) unter Bedingungen hydrolytisch zur 20-Aminoverbindung spaltbar sind, die eine Mitreaktion anderer an sich reaktiver Stellen des Steroidgerüstes, insbesondere im A-Ring des Systems, ausschließen. Das entsprechende Methylcarbamat oder in noch stärkerem Maße die entsprechenden Carbamate mit C-2- oder C-3-Alkoholen fordern zur hydrolytischen Spaltung agressive saure Verfahrensbedingungen. Ihre Spaltung gelingt befriedigend beispielsweise mit konzentrierter Salzsäure, die zusätzlich mit gasförmigem HCl gesättigt ist. Unter solchen Bedingungen reagiert jedoch der A-Ring des Steroidsystemes, sei es durch Anlagerung von Halogen bzw. Halogenwasserstoff und/oder durch Aromatisierung. Das 20-tert.-Butylcarbamat läßt sich jedoch schon mit vergleichsweise schwachen Säuren, wie Essigsäure, Oxalsäure, oder Mineralsäuren, wie Salzsäure, einwandfrei zur 20-Aminoverbindung hydrolysieren. In gleicher Weise kann das 20-Isocyanat mit solchen wäßrigen Säuren, beispielsweise wäßriger Essigsäure, zur 20-Aminoverbindung gespalten werden, ohne daß andere unerwünschte Änderungen der Steroidstruktur eintreten.

Die Isocyanat- bzw. tert.-Butylcarbamatspaltung kann im Temperaturbereich von 0 bis 100°C, vorzugsweise im Temperaturbereich von etwa 10 bis 90°C, durchgeführt werden. Zweckmäßigerweise arbeitet man bei Temperaturen von 50 bis 80°C. Es kann zweckmäßig sein, die Umsetzung unter Stickstoff durchzuführen. Die eingesetzte Wassermenge entspricht wenigstens der stöchiometrisch erforderlichen Menge, vorzugsweise wird Wasser im Überschuß eingesetzt. Gleichwohl ist es in der Regel bevorzugt, konzentrierte wäßrige Säuren, wie konzentrierte Salzsäure bzw. konzentrierte wäßrige Essigsäure, als Reaktionsmedium zu verwenden.

Die erfindungsgemäße 20-Amino-Verbindung fällt bei der sauren Hydrolyse als Salz der zur Hydrolyse eingesetzten Säure(n) an. Die Umwandlung dieses Salzes in die freie Base ist in an sich bekannter Weise durch Behandlung mit starken Basen möglich. Ebenso können die Salze bzw. die Base in beliebige andere Salze umgewandelt werden.

Die Umwandlung der 20-Aminogruppen in die 20-Oxogruppe — d. h. die Gewinnung von Dehydroprogesteron aus der erfindungsgemäßen Verbindung — kann dann beispielsweise durch Reaktion der freien Base mit einem 3,5-disubstituierten o-Chinon zum entsprechenden Steroid-20-Anil mit anschließender Hydrolyse dieses Anils erfolgen, wie es in der parallelen Europäischen Patentanmeldung 81101115.4/34365 (»Verfahren zur Herstellung von 3,20-Dioxo-pregn-4-en und/oder 3,20-Dioxo-pregna-1,4-dien« mit U. S. Priorität vom 19. Februar 1980, Aktenzeichen 122 396) beschrieben ist.

Aus dem Stand der Technik zur chemischen Transformation von Steroidverbindungen wird verwiesen auf Canadian Journal of Chemistry, Band 34 (1956), S. 982—990, sowie Helvetica Chimica Acta, Band XXXII, Teil VI (1949), No. 255, S. 1922—1933, sowie a. a. O. Band XXXII, Teil V (1949), No. 233, S. 1764—1769. Verwiesen wird weiterhin auf JACS, Band LXX (1948), No. 3, S. 887—892, sowie Helvetica Chimica Acta, Band XXXII, Teil V (1949), No. 232, S. 1758—1763. Verwiesen sei schließlich auch noch auf die US-PS 3 519 658.

Beispiel

Das erfindungsgemäße Steroid-20-Amin wird aus dem 3-Oxo-pregna-1,4-dien-20-isocyanat durch Hydrolyse mit wäßriger Essigsäure bei 65° C mit anschließender Wasserdampfdestillation in Form des Acetatsalzes erhalten, das durch Behandlung mit verdünnter Base (Natriumhydroxid) zum freien Amin umgewandelt werden kann. Im einzelnen wird wie folgt gearbeitet:

Eingesetzt werden 7,5 mmol (2,54 g) der entsprechenden Isocyanatverbindung, 0,89 mol (50,8 ml) konzentrierte Essigsäure, 0,57 mol (10,2 ml) Wasser und 60,3 mmol (5 ml) konzentrierte Salzsäure.

Das Isocyanat wird in der wasserhaltigen Essigsäure für den Zeitraum von 2 Stunden auf 65° C erhitzt. Die Lösung wird dann am Rückfluß erhitzt und der Wasserdampfdestillation unterworfen, bis 300 ml Destillat übergegangen sind. Dann wird die Salzsäure zugesetzt und die Lösung weiterhin mit Wasserdampf destilliert, bis weitere 100 ml Destillat übergegangen sind.

Der Reaktionsrückstand wird auf 20° C gekühlt, mit Kochsalz gesättigt und mehrfach mit Methylenchlorid extrahiert. Die Extraktphase wird gewaschen und zur Trockne eingedampft. Es werden 1,31 g des Aminsalzes erhalten. Dünnschicht-Chromatographie und Gas-Chromatographie belegen, daß das 20-Amino-3-oxo-pregna-1,4-dien-hydrochlorid als Reaktionsprodukt entstanden ist.

Geht man vom 20-tert.-Butylcarbamat als Ausgangsmaterial aus, so kann dieses mit wasserhaltiger Essigsäure oder mit wäßriger Salzsäure in vergleichbarer Weise zum 20-Aminsalz gespalten werden.

Analysenwerte
$\Delta^{1,4}$-BNC-Amin
Fp 168 – 170° C

Elementaranalyse

| | | | |
|---|---|---|---|
| Berechnet: | C 80,46 | H 9,97 | N 4,47 |
| Gefunden: | C 80,5 | H 10,0 | N 4,40 |

$^1$H-NMR-Spektrum (80 MHZ, $CDCl_3$, $\delta$-Werte):

| | | | |
|---|---|---|---|
| 18-$CH_3$ | 0,74 ppm, | s | |
| 19-$CH_3$ | 1,22 ppm, | s | |
| 21-$CH_3$ | 1,10 ppm, | d | (J = 8 Hz) |
| 20-CH | 2,80 ppm, | m | |

- olefinische Protonen: ABC-System mit Signalen bei: 6,06; 6,13; 6,16; 6,26; 6,28; 6,98; 7,11 ppm
- die übrigen Protonen liefern Signale im erwarteten Bereich

## Patentansprüche

1. 20-Amino-3-oxo-pregna-1,4-dien und seine Salze mit anorganischen oder organischen Säuren.
2. Verfahren zur Herstellung von 20-Amino-3-oxo-pregna-1,4-dien bzw. seinen Salzen, dadurch gekennzeichnet, daß man 3-Oxo-pregna-1,4-dien-20-isocyanat und/oder 3-Oxo-pregna-1,4-dien-20-tert.-butylcarbamat hydrolytisch spaltet.
3. Verfahren nach Anspruch 2, dadurch gekennzeichnet, daß man mit wäßrigen Säuren, insbesondere wäßriger Essigsäure, arbeitet und gewünschtenfalls das gebildete Aminsalz durch Behandlung mit einer starken Base in das freie Amin umwandelt.

## Claims

1. 20-Amino-3-oxo-pregna-1,4-diene and its salts with inorganic and organic acids.
2. A process for preparing 20-amino-3-oxo-pregna-1,4-diene and its salts, respectively, characterized in that 3-oxo-pregna-1,4-diene-20-isocyanate and/or 3-oxo-pregna-1,4-diene-20-tert-butylcarbamate are hydrolytically decomposed.
3. The process according to claim 2, characterized in that it is effected by using aqueous acids, more particularly aqueous acetic acid, and that the amine salt having been formed is converted into the free amine by a strong base if desired.

## Revendications

1. 20-Amino-3-oxo-prégna-1,4-diène et ses sels d'acides inorganiques ou organiques.
2. Procédé pour la fabrication de 20-amino-3-oxo-prégna-1,4-diène ou de ses sels, caractérisé en ce

que l'on dissocie par hydrolyse le 3-oxo-prégna-1,4-diène-20-isocyanate et/ou le 3-oxo-prégna-1,4-diène-20-carbamate de tert-butyle.

3. Procédé selon la revendication 2, caractérisé en ce que l'on opère avec des acides aqueux, en particulier l'acide acétique aqueux, et on transforme, si on le désire, le sel d'amine formé en amine libre par traitement par une base forte.